(19) Europäisches Patentamt
European Patent Office
Office européen des brevets

(11) Publication number: **0 231 997 B1**

# (12) EUROPEAN PATENT SPECIFICATION

(45) Date of publication of patent specification: **05.08.92** (51) Int. Cl.$^5$: **A61K 7/08**

(21) Application number: **87300074.9**

(22) Date of filing: **07.01.87**

## (54) Shampoo.

(30) Priority: **09.01.86 GB 8600496**

(43) Date of publication of application:
**12.08.87 Bulletin 87/33**

(45) Publication of the grant of the patent:
**05.08.92 Bulletin 92/32**

(84) Designated Contracting States:
**AT BE CH DE ES FR GB GR IT LI NL SE**

(56) References cited:
**EP-A- 0 170 927**
**FR-A- 2 358 877**
**US-A- 3 996 146**
**US-A- 4 329 336**

**CHEMICAL ABSTRACTS, vol. 96, no. 10, 8th March 1982, page 367, abstract no. 74500s, Columbus, Ohio, US; & JP-A-81 122 900 (JOHNSON CO., LTD) 26-09-1981**

(73) Proprietor: **UNILEVER PLC**
**Unilever House Blackfriars P.O. Box 68**
**London EC4P 4BO(GB)**
(84) Designated Contracting States:
**GB**

(73) Proprietor: **UNILEVER N.V.**
**Burgemeester s'Jacobplein 1 P.O. Box 760**
**NL-3000 DK Rotterdam(NL)**
(84) Designated Contracting States:
**BE CH DE ES FR GR IT LI NL SE AT**

(72) Inventor: **Reid, Euan Stuart**
**4 Meadway**
**Bebington Wirral L62 2AR(GB)**

(74) Representative: **Tonge, Robert James et al**
**UNILEVER PLC Patent Division Colworth House Sharnbrook**
**GB-Bedford MK44 1LO(GB)**

EP 0 231 997 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### TECHNICAL FIELD

The invention relates to shampoos which, when employed in the washing of hair, provide an improvement to the quality of the foam normally resulting from the presence of detergent active compound, but without increasing the difficulty of rinsing the foam from the hair after shampooing is completed.

### BACKGROUND

Certain polymers can be incorporated in aqueous liquid shampoos in order to increase the viscosity of the shampoo, to improve the quality of the foam or to impart conditioning benefits to the hair when washed with such shampoos.

For example, in "Water-soluble Polyacrylamides in Cosmetics" by Isacoff et al, published in Drug & Cosmetic Industry, October 1978 at page 48, there is described an aqueous shampoo containing the polyacrylamide known as Gelamide 250 at a concentration of 0.5%. This polyacrylamide is stated to have a molecular weight of approximately $5 \times 10^6$ to $6 \times 10^6$.

Also, US patent 4,329,336 (Colgate Palmolive Co.) describes the use of from 0.05 to 1% by weight of a water-soluble polyacrylamide having an average molecular weight of $1 \times 10^5$ to $3 \times 10^6$, as a thickening agent in an aqueous antidandruff nonionic shampoo.

FR-A-2,358,877 (Colgate Palmolive Co.) describes the use of from 0.05 to 1% by weight of polyacrylamide of average molecular weight preferably $1 \times 10^5$ to $3 \times 10^6$ in an aqueous nonionic shampoo. This composition may contain polymeric thickeners.

Also, Japanese patent Kokai No. 56/122900 (Johnson KK) concerns an improvement to the quality of the foam produced by shampoo containing from 0.0001 to 3% by weight of a polyacrylamide. Optionally, polyethylene oxide and/or sodium polyacrylate can also be present.

There is a problem common to the use of these and other shampoos, in that rinsing residual foam from the hair can be difficult. It can accordingly be necessary to rinse the hair at least twice with water or continuously, for example under a shower, for an inconvenient length of time, before the hair can be pronounced to be free from foam.

We have now found that the inconvenience attributable to the use of shampoos such as those referred to hereinbefore, can be avoided by employing a shampoo which contains a low concentration of a very high molecular weight polyacrylamide and a second polymer which is present as a thickener or a conditioner. The combined use of the polyacrylamide and the second polymer produces an unusually rich creamy foam which improves the distribution of the shampoo throughout the hair, thereby enhancing the cleaning of the hair, and also helps to prevent hair damage during shampooing. The feel of this rich creamy foam provides a pleasurable sensation recognisable by the user; the foam is moreover easy to rinse from the hair.

### DEFINITION OF THE INVENTION

Accordingly, the invention provides an aqueous shampoo comprising, in addition to water:

a) from 2 to 40% by weight of detergent active compound;

b) from 0.001 to 0.5% by weight of a polyacrylamide having a molecular weight of at least $1 \times 10^5$; and

c) a second polymer chosen from cationic polymers, nonionic polysaccharides and mixtures thereof,

with the proviso that if the polyacrylamide has a molecular weight in the range from $1 \times 10^5$ to $8 \times 10^6$, then the second polymer is a polymer or mixture of polymers chosen from: polygalactomannan gums, polyglycol-polyamine condensation resins, poly(dimethyldiallyl ammonium choride) polymers, hydroxyethylcelluloses, hydroxypropylmethyl celluloses, cationic cellulose ether polymers having the structure:

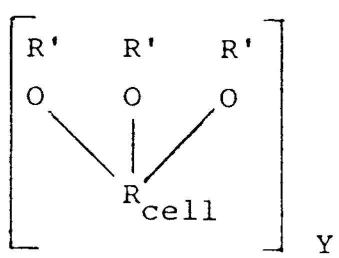

wherein $R_{cell}$ represents the residue of an anhydroglucose unit, Y is an integer of from 50 to 20,000 and wherein each R' individually represents a substituent group of the following general formula:

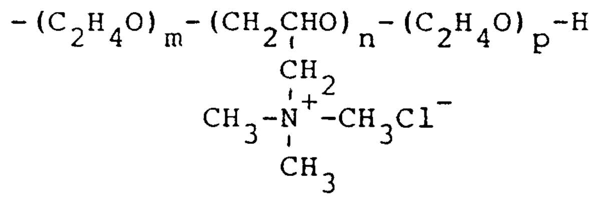

where m is O, or an integer of from 1 to 10, n is O, or an integer of from 1 to 3, and p is O, or an integer of from 1 to 10, the average value of n per anhydroglucose unit being from 0.35 to 0.45 and the average value of the sum of m and p, per anhydroglucose unit being from 1 to 2;

the amount of the second polymer being from 0.05 to 5% by weight if the second polymer is a polyglycol-polyamine condensation resin, from 0.05 to 3% by weight if the second polymer is poly-(dimethylkdiallyl ammonium chloride) and otherwise from 0.05 to 2% by weight.

## DISCLOSURE OF THE INVENTION

### The Detergent Active Compound

The shampoo according to the invention comprises detergent active compound chosen from anionic, nonionic and amphoteric detergent active compounds or mixtures thereof. Preferably, the shampoo comprises an anionic detergent active compound, optionally together with one or more further detergent active compounds chosen from nonionic and amphoteric detergent active compounds and mixtures thereof.

The anionic detergent active compound is most preferably an alkyl sulphate, an alkyl ether sulphate or mixtures thereof. Commonly used anionic detergent active compounds of these kinds are $C_{10}$ to $C_{18}$ alkyl sulphates, and $C_{10}$ to $C_{18}$ alkyl ether sulphates containing 1 to 5, preferably 2 or 3 moles of ethylene oxide. These detergents are generally employed in the form of their sodium, potassium, ammonium or mono-, di- or triethanolamine salts. Examples of these detergents are sodium lauryl sulphate, ammonium lauryl sulphate, mono-, di- and tri- ethanolammonium lauryl sulphates, sodium lauryl ether sulphate (2EO), sodium lauryl ether sulphate (3EO), potassium lauryl ether sulphate (2EO) and ammonium lauryl ether sulphate (3EO).

Further suitable anionic detergent active compounds include dialkyl sulphosuccinates having the structure:

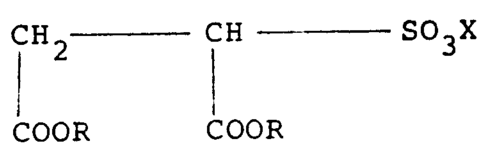

where R represents the same or different straight chain or branched chain alkyl groups having from 4 to 12 carbon atoms;
X is a solubilising cation chosen from alkali metal, ammonium, substituted ammonium and magnesium.

Particularly preferred dialkyl sulphosuccinates include those where R represents $C_6$ and/or $C_8$ alkyl groups.

Still further suitable anionic detergent active compounds include α-olefin sulphonates, alkyl sarcosinates, alkyl monoglyceride sulphates, alkyl monoglyceride sulphonates, alkyl benzene sulphonates, monoalkylether sulphosuccinates, alkyl ether carboxylates, acyl isethionates and acyl methyl taurides.

An example of a nonionic detergent active compound is the reaction product of a sorbitan monolaurate or a sorbitan monococoate with from 20 to 80 moles of ethylene oxide and a $C_8$ to $C_{18}$ ethoxylated fatty alcohol having from 10 to 50 moles of ethylene oxide.

Examples of amphoteric detergent active compounds include amine oxides, betaines, sulphobetaines, and imidazolines.

The amount of detergent active compound present in the shampoo of the invention will depend on the

product form of the shampoo. For example, when the shampoo is a propellant-free liquid or gel intended to be poured, pumped or otherwise dispensed from a bottle or similar container or from a sachet, then the shampoo will generally contain from 2 to 30%, preferably from 10 to 20% by weight of detergent active compound. When, however, the shampoo is in the form of a liquid or gel containing a gaseous liquefiable propellant, intended to be dispensed from an aerosol can, generally as a mousse, then the shampoo concentrate, that is that part of the product without propellant, will generally contain from 2 to 40%, preferably from 10 to 30% by weight of detergent active compound.

Having regard to the product form of the shampoo, it can be stated generally that shampoos containing less than about 2% by weight of detergent active compound are likely in use to produce insufficient lather to clean the hair efficiently, while shampoos containing more than 40% by weight of detergent active compound may be too thick to apply conveniently to the hair and may also degrease the hair to an undesirably excessive degree.

The individual amounts of anionic detergent active compound, and nonionic and amphoteric detergent active compound, when present, will fall within limits for total detergent active compound as defined herein. Accordingly, when only anionic detergent active compound is present, then it will comprise from 2 to 40%, preferably from 4 to 20% by weight of the shampoo. When a nonionic detergent active compound is present, then it will normally comprise up to 20%, preferably from 1 to 10% by weight of the shampoo. When an amphoteric detergent active compound is present, then it will normally comprise up to 20%, preferably from 1 to 10% by weight of the shampoo.

## The Polyacrylamide

The polyacrylamide employed in the shampoo according to the invention is an acrylamide polymer which can be a homopolymer of acrylamide, or a copolymer of acrylamide, with up to 15% by weight of other suitable monomers such as methacrylamide, acrylic acid and methacrylic acids and the alkali metal salts and lower alkyl esters thereof and vinyl alkyl ethers, provided each such copolymer is characterised by water solubility, molecular weight and viscosity properties as described hereinafter. In the copolymers of acrylamide with acrylic or methacrylic acid or their alkali metal salts, higher proportions of the second ingredient to the acrylamide ingredients may be employed, and the expression "polyacrylamide" accordingly includes copolymers of acrylamide with up to 50 mole per cent of acrylic or methacrylic acid or an alkali metal salt thereof.

Water-soluble polyacrylamides are usually characterised by a greater or lesser degree of hydrolysis due to the presence of some free carboxyl groups. This condition is dependent upon the method of manufacture of the polymer, the presence or absence of acrylic or methacrylic acid in the starting monomer mixture, and conditions of storage of the polymer. The polymer products appear to be equivalent whether the carboxyls result from copolymerisation of acrylamide with acrylic or methacrylic acid, or from hydrolysis of amide or ester groups subsequent to polymerisation. In the practice of the present invention, the polyacrylamides are those not having more than about 10% of carboxyl moieties based on the total of carboxyl and amide moieties in the polymer.

The polyacrylamides are water-soluble and have a low degree of cross-linking. In order to obtain the desired enhancement of the foam obtained when the shampoo is used to wash the hair, i.e. to provide a foam with a pleasant creamy feel, it is necessary to employ high molecular weight polyacrylamides, that is polymers characterised by a viscosity of at least 0.004 PaS for a 0.5% by weight solution of the polymer in an aqueous 4% by weight sodium chloride solution adjusted to a pH of 5 to 6 at a temperature of 25°C, such viscosity being measured with a Brookfield viscometer.

The polyacrylamide having a viscosity of at least 0.004 Pa.s as defined above will have a molecular weight of at least $1 \times 10^5$, preferably from $1 \times 10^6$ to $1 \times 10^8$.

Particularly preferred examples of commercially available polyacrylamides are Magnafloc LT20 (supplied as a powder), having a molecular weight of from $8 \times 10^6$ to $12 \times 10^6$, available from Allied Colloids Limited, and Floc Aid 400 (supplied as a 28% by weight dispersion in liquid hydrocarbon), having a molecular weight of $15 \times 10^6$, available from National Adhesives and Resins Limited.

The shampoo will generally contain from 0.001 to 0.5%, preferably from 0.005 to 0.2% by weight of the polyacrylamide.

Shampoos containing less than about 0.001% by weight of polyacrylamide are unlikely in use to enhance the creaminess of the lather obtained when the hair is washed with the shampoo. Shampoos containing more than 0.5% by weight of the polyacrylamide present difficulties in rinsing away the foam that is likely to be produced.

The Second Polymer

The shampoo according to the invention will also comprise a second polymer, which is chosen from cationic polymers, nonionic polysaccharide polymers or mixtures thereof.

When the second polymer is a cationic polymer, its function in the shampoo will generally be that of a hair conditioner. When the second polymer is a nonionic polysaccharide polymer, its function in the shampoo will generally be that of a thickener.

Particularly preferred second polymers, other than polyacrylamide, include polygalactomannan gums, cellulosic polymers, polyglycol-polyamine condensation resins, poly(dimethyldialkylammonium chloride) polymers or related cationic polymers, and mixtures thereof.

The polygalactomannan gums and certain derivatives thereof, which can be employed as the second polymer in the shampoo according to the invention, occurs naturally as guar gum, the principal component of the seed of the guar plant Cyamopsis tetragonalobus.

Examples of polygalactomannan gums include hydroxypropyltrimethyl ammonium guar gums, which are cationic in character, and hydroxypropyl guar gums, which are polysaccharides and nonionic in character. These polygalactomannan gums are available from Celanese-Stein Hall under the trade name JAGUAR.

When the polygalactomannan gum derivative is present in the shampoo of the invention, it should form from 0.05 to 2%, preferably not over 1% by weight of the shampoo.

The cellulosic polymers which can be employed as the second polymer in the shampoo according to the invention include cationic cellulose ether polymers having the structure:

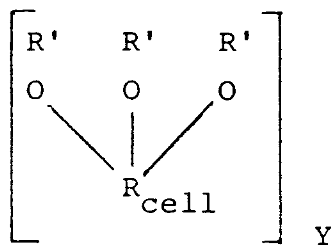

wherein $R_{cell}$ represents the residue or an anhydroglucose unit, Y is an integer of from 50 to 20,000 and wherein each R' individually represents a substituent group of the following general formula:

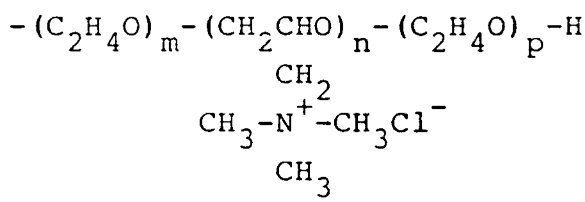

where m is O, or an integer of from 1 to 10, n is O, or an integer of from 1 to 3, and p is O, or an integer of from 1 to 10. The average values per anhydroglucose unit are: n from 0.35 to 0.45 and the sum of m + p is from 1 to 2. The polymers have molecular weights between about 100,000 and 3,000,000. The viscosity of the preferred cationic cellulose ethers is from 50 to 35,000 mPa.s (50 to 35,000 cps) measured according to ASTM D 2364-65 at 25 °C in a 2% by weight aqueous solution (Brookfield viscometer LVF, 30 rpm, spindle No.2). Especially suitable are the cationic cellulosic derivatives sold by the Union Carbide Corporation under the trade names "JR 125", "JR 400", and "JR 30M" signifying a polymer of the type described having viscosities of 125 mPa.s (cps), 400 mPa.s (cps), and 30,000 mPa.s (cps), respectively.

Suitable cellulosic polymers also include nonionic polymers such as hydroxyethyl celluloses available from Hercules under the trade name NATROSOL, and hydroxypropylmethyl celluloses available from Dow under the trade name METHOCEL.

When the cellulosic polymer is present in the shampoo of the invention, it should form from 0.05 to 2%, preferably from 0.1 to 1% by weight of the shampoo.

The polyglycol-polyamine condensation resin polymers which can be employed as the second polymer in the shampoo according to the invention are hardenable polycondensation products produced by reacting

a polyamine compound having from 2 to 10 carbon atoms with an ether of polyoxyalkylene glycol having terminal halogen or hydroxyl groups and having from 2 to 4 carbon atoms in the alkylene units thereof, said polyamine reaction compounds having more than one hydrogen atom attached to a nitrogen atom and being further reacted with a bifunctional aliphatic compound having functional groups chosen from epoxide and α-halo-β-hydroxyalkyl.

Preferably, the polycondensation product is a reaction product chosen from:

i) the reaction product of dipropylenetriamine, bis-chlorohydrin ether of polyoxyethylene glycol having an average molecular weight of about 1,000 and epichlorohydrin;

ii) the reaction product of dipropylenetriamine, bis-chlorohydrin ether of polyoxyetheylene glycol having an average molecular weight of about 1,000, and bis-chlorohydrin ether of polyoxyethylene glycol having an average molecular weight of about 600;

iii) the reaction product of diethylenetriamine, ethoxylated ethylene chlorohydrin, and epichlorohydrin;

iv) the reaction product of dipropylenetriamine, ethoxylated glycerin chlorohydrin ether, and epichlorohydrin;

v) the reaction product of triethylenetetramine, bis-chlorohydrin ether of polyoxyethylene glycol having an average molecular weight of about 1,000 and bis-chlorohydrin ether of polyoxyethylene glycol having an average molecular weight of about 600;

vi) the reaction product of dipropylenetriamine and bis-chlorohydrin ether of polyoxyethylene glycol having an average molecular weight of about 600; or

vii) the reaction product of dipropylenetriamine and bis-chlorohydrin ether of polyoxyethylene glycol having an average molecular weight of about 200, wherein the ratio of chlorine atoms to amino hydrogen atoms in the reaction products is 4:5 to 7:5.

The preferred polyglycol-polyamine condensation resin for use in shampoos according to the invention is that which is available commercially under the trade name POLYQUART H from Henkel & Cie GmbH, as a 50% aqueous solution. POLYQUART H is a trade mark.

When the polyglycol-polyamine condensation resin is present in the shampoo of the invention, it should form from 0.1 to 5%, preferably from 0.2 to 3% by weight of the shampoo.

The poly(dimethyldiallylammonium chloride) polymers which can be employed as the second polymer in the shampoo according to the invention are cationic polymers and are available commercially from the Merck Chemical Division of Merck & Co. Inc., USA, under the trade name MERQUAT

Cationic polymers related to poly(dimethyldiallylammonium chloride) include poly(dimethylbutenyl ammonium chloride)- α,ω-bis(triethanolammonium chloride) which is commercially available from the Onyx Chemical Co., USA under the trade name ONAMER M. Other suitable cationic polymers include poly-(dipropyldiallyammonium chloride), poly(methyl-β-propaniodiallylammonium chloride), poly-(diallylpiperidinium chloride), poly(vinyl pyridinium chloride), quaternised poly(vinyl alcohol) and quaternised poly (dimethylaminoethylmethacrylate). Further suitable cationic polymers are those known as Polymer QR 686, an imidazoline acetate derivative available from Rohm & Haas, and CARTARETIN K, a cross-linked polyamidepolyamine available from Sandoz.

When the poly(dimethylallylammonium chloride) polymers or other cationic polymers referred to herein are present in the shampoo of the invention, they should form preferably from 0.05 to 3%, preferably not over 2%, more preferably not over 1% by weight of the shampoo.

It is to be understood that the second polymers referred to herein by way of example do not represent an exhaustive list of second polymers, and that other cationic polymers and nonionic polysaccharide polymers are suitable for use in the performance of the invention.

## Water

The shampoo according to the invention is an aqueous shampoo and will accordingly normally contain mot more than 94.94%, normally from 50 to 90%, and preferably from 65 to 80% by weight of water.

## Optional ingredients

The shampoo according to the invention can contain other ingredients which are conventionally employed in the manufacture of shampoos.

An example of an optional ingredient is a phosphate ester having the structure:

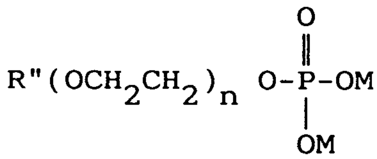 (7)

and/or a diester of the formula

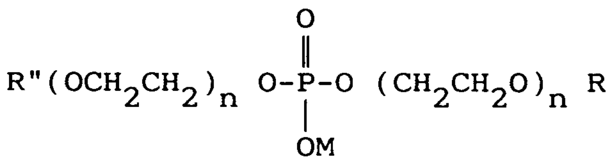 (8)

where

- R'' is an hydrocarbon group having from 8 to 22 carbon atoms;
- n is O or an integer of from 1 to 10; and
- M is hydrogen or an alkali metal, ammonium or amine salt forming group.

The hydrocarbon group R'' is preferably one chosen from alkyl, alkenyl, cycloalkyl or cycloalkenyl groups.

When the group M is an alkali metal, it is preferably sodium or potassium, and when the group M is an amine salt-forming group it can be a substituted ammonium group, particularly lower alkyl and hydroxy lower alkyl groups, especially mono-, di- and tri- ethyl, propyl, hydroxyethyl and hydroxypropyl-substituted ammonium groups.

The phospate ester is usually produced commercially as a mixture of the mono- and di- esters defined above. These are available commercially under various trade names, for example, BEROL, BRIPHOS, CRODAFOS and HOSTAPHAT.

Specific examples of phosphate esters include:

i) BEROL 729 having an alkyl chain length of from 16 to 18 carbon atoms and containing a series of 4 ethylene oxide units;

ii) BRIPHOS L2D in which the alkyl chains are lauryl groups and it contains series of 2 ethylene oxide units;

iii) BRIPHOS 03D and CRODAFOS N3N in which the alkyl groups are oleyl and the ethylene oxide groups comprise 3 units;

iv) HOSTAPHAT KO 300N in which mono-, di- and triphosphoric esters of oleic acid are present.

For mono- and di-ester mixtures, the weight ratio of mono-ester to di-ester may vary, typically from 1:10 to 10:1. Preferred phosphate esters are those in which n is 0 or an integer of from 1 to 6, in the structural formulae (7) and (8) set out hereinbefore.

The phosphate ester can optionally be included in the shampoo in an amount of from 0.1% to 3%, from 0.2% to 2.5% by weight of the shampoo.

The shampoo of the invention can also include appropriate amounts of other conventional ingredients which can be employed in shampoos; these include for example a foam booster, a thickener, an opacifier, a perfume, a colouring agent, a preservative, a protein and an agent for adjusting pH, usually to a value in the range of from 4 to 9, preferably from 5.5 to 7.5.

The shampoo can be formulated as a clear shampoo or as an opaque shampoo, depending upon consumer preference and available packaging.

## PROCESS FOR THE MANUFACTURE OF SHAMPOO

The invention also provides a process for the manufacture of shampoos according to the invention which comprises the steps of incorporating together in aqueous solution, dispersion or suspension, the detergent active compound, the polyacrylamide and the second polymer as hereindefined, together with optional ingredients as desired.

It is particularly important to ensure that the polyacrylamide is uniformly dispersed in the shampoo before it is thickened, so as to avoid the formation of lumps of this ingredient which the consumer would find unacceptable. Ideally, the polyacrylamide is dispersed in non-aqueous ingredients such as perfume, or

predispersed in hydrocarbon in the form as supplied by the manufacturer. It is also possible to disperse the polyacrylamide in at least a portion of the water employed in the manufacture of the shampoo, provided that care is taken to avoid the formation of aggregates or lumps of the polyacrylamide which would be difficult or impossible to disperse later in the manufacturing process.

According to one preferred embodiment of the invention, the process for the manufacture of the shampoo comprises the steps of:

(i ) dispersing the polyacrylamide in a non-aqueous liquid chosen from perfume oils, hydrocarbon and mixtures thereof, and

(ii) subsequently incorporating into the dispersion, the detergent active compound, the second polymer and water to form the shampoo.

According to a second preferred embodiment of the invention, the process for the manufacture of the shampoo comprises the steps of:

(i ) dispersing the polyacrylamide in a portion of water to form an aqueous solution of the polyacrylamide, and

(ii) subsequently incorporating into the solution the detergent active compound, the second polymer and the remaining water to form the shampoo.

USE OF THE SHAMPOO

The shampoo according to the invention can be employed in the washing and conditioning of human hair according to conventional shampooing techniques. The shampooing of hair can accordingly include the steps of pre-wetting the hair with water, adding to the hair an appropriate quantity of from about 1 to 10 ml of the shampoo, massaging the hair in order to provide a creamy lather thereby to enhance the distribution of the shampoo on the hair and to improve the cleaning thereof, and rinsing the hair and subsequently styling and drying the hair in a desired fashion.

EVIDENCE TO DEMONSTRATE ENHANCED FOAM CREAMINESS WITHOUT ADVERSELY AFFECTING THE EASE OF RINSING OF FOAM FROM SHAMPOOED HAIR

Test Method

The benefits to be derived from use of the shampoo according to the invention were demonstrated using in-vivo tests where panellists assess the characteristics of shampoo foams while washing their own hair. Each test consists of a paired comparison of two products using 40 panellists who wash their hair under standard conditions (at a sink with a shower attachment). The first product is applied, lathered up and rinsed off, then the second product is applied, lathered up and again rinsed off. While doing this the panellists assess the foams for amount, creaminess and ease of rinsing. The dosage of each product is 6g and the order of applying the two products is balanced across the panel. This methods allows for sensitive comparison of different formulations under realistic conditions using relatively small panels. In principal any sensory scaling method may be used for such a test but we use the ratio scaling method, Magnitude Estimation, in preference.

Demonstration of the benefits of the Invention

The benefits of the invention were demonstrated by a series of paired comparison tests. Each test consisted of a comparison of a simple shampoo base with and without added polymer or combination of polymers. The formulation of the simple base shampoo was as follows:

| | % weight |
|---|---|
| Sodium lauryl ether sulphate 2EO (SLES 2EO) | 12.0 |
| Coconut diethanolamide (CDE) | 2.0 |

Tests were conducted using a high molecular weight polyacrylamide at different levels, a cationic guar gum at different levels and finally a combination of these two polymers. The results of these tests are given in Table 1. The figures shown are the differences in the mean Magnitude Estimates across the panel for each attribute assessed. Positive numbers imply that the polymer containing shampoo has more of that attribute while negative numbers imply that the polymer containing shampoo has less of that attribute.

TABLE 1

PANELLIST ASSESSED COMPARISONS OF A SIMPLE SHAMPOO WITH AND WITHOUT POLYMER

| POLYMER | LEVEL (%) | CREAMINESS OF FOAM | EASE OF RINSING |
|---|---|---|---|
| Polyacrylamide | ( 0.05 | 8 | -1 |
| (M.wt. 8 x $10^6$) | ( 0.1 | 15 | -2 |
| [MAGNA FLOC LT20] | ( 0.2 | 18 | -12 |
| Second polymer: | ( 0.1 | 7 | -2 |
| Cationic guar gum | ( 0.3 | 15 | -4 |
| [JAGUAR C13S]* | ( 0.5 | 17 | -8 |
| MAGNA FLOC LT20: | 0.1 ) | | |
| plus | + ) | 31 | -3 |
| JAGUAR C13S: | 0.3 ) | | |

*Hydroxypropyltrimethyl ammonium guar gum, where the degree of substitution of the cationic groups is 0.13 .

These results illustrate how increasing levels of either polymer give creamier foams but there is a limiting level above which the ease of rinsing is adversely effected. The combination of polymers however gives a much greater foam creaminess without dramatically decreasing the ease of rinsing.

EXAMPLES OF THE INVENTION

The invention is illustrated by the following examples of shampoo formulations:

| | % w/w |
|---|---|
| <u>Example 1</u> | |
| SLES 2EO | 12 |
| CDE | 2 |
| JAGUAR C13S | 0.3 |
| MAGNA FLOC LT20 | 0.1 |
| Perfume | 0.5 |
| Water | to 100 |

| | |
|---|---|
| <u>Example 2</u> | |
| SLES 2EO | 16 |
| Coconut monoisopropanolamide (CIS) | 3 |
| JAGUAR C13S | 0.3 |
| FLOC AID 400* | 0.2 |
| Perfume | 0.5 |
| Water | to 100 |

* Polyacrylamide of M.wt $15 \times 10^6$

| | |
|---|---|
| <u>Example 3</u> | |
| SLES 2EO | 12 |
| CDE | 2 |
| JAGUAR C13S | 0.3 |
| BRIPHOS 03D | 1.5 |
| FLOC AID 400 | 0.2 |
| JAGUAR HP60+ | 0.3 |
| Perfume | 0.5 |
| Water | to 100 |

+ Hydroxypropyl guar gum, where the degree of substitution of the nonionic groups is 0.6

| Example 4 | % w/w |
|---|---|
| SLES 2EO | 12 |
| CIS | 3 |
| POLYMER JR 400 | 0.5 |
| FLOC AID 400 | 0.1 |
| Perfume | 0.5 |
| Water | to 100 |

| Example 5 | |
|---|---|
| Ammonium lauryl sulphate (ALS) | 16 |
| CIS | 3 |
| JAGUAR C13S | 0.3 |
| FLOC AID 400 | 0.05 |
| Perfume | 0.5 |
| Water | to 100 |

| Example 6 | |
|---|---|
| Di-alkyl sulphosuccinate (DIASS) | 12 |
| JAGUAR HP60 | 0.3 |
| FLOC AID 400 | 0.1 |
| Perfume | 0.5 |
| Water | to 100 |

| Example 7 | |
|---|---|
| SLES 2EO | 15 |
| CDE | 2 |
| MERQUAT 550* | 3.0 |
| MAGNA FLOC LT20 | 0.08 |
| Perfume | 0.5 |
| Water | to 100 |

* Poly(dimethyldiallylammonium chloride) : supplied as solution containing 8% by weight solids.

| Example 8 | % w/w |
|---|---|
| ALS | 14 |
| CIS | 3 |
| POLYQUART H | 1 |
| FLOC AID 400 | 0.1 |
| Water | to 100 |

| Example 9 | |
|---|---|
| SLES 2EO | 10 |
| CDE | 2 |
| NATROSOL 250 HR | 1 |
| FLOC AID 400 | 0.1 |
| Water | to 100 |

| Example 10 | |
|---|---|
| SLES 2EO | 13.5 |
| CDE | 2.5 |
| METHOCEL E4M | 0.8 |
| FLOC AID 400 | 0.15 |
| Water | to 100 |

| Example 11 | |
|---|---|
| Lauryl dimethyl amine oxide | 10 |
| NATROSOL 250HR | 1 |
| FLOC AID 400 | 0.1 |
| Perfume | 0.5 |
| Water | to 100 |

| Example 12 | |
|---|---|
| Lauryl alcohol ethoxylate (12EO) | 10 |
| NATROSOL 250 HR | 1 |
| FLOC AID 400 | 0.1 |
| Perfume | 0.5 |
| Water | to 100 |

Certain of the shampoos illustrated in the foregoing examples were subjectively assessed for creaminess of foam and ease of rinsing by panellists, in accordance with the Test Method as hereinbefore described.

The results, which should be interpreted in the manner employed for Table 1, are set out below in Table 2.

TABLE 2

| PANELLIST ASSESSED COMPARISIONS OF SELECTED EXAMPLE SHAMPOOS - WITH AND WITHOUT THE POLYACRYLAMIDE INGREDIENT | | |
|---|---|---|
| Example | creaminess of foam | Ease of rinsing |
| 1 | +12 | 0 |
| 2 | +13 | 0 |
| 3 | +11 | -1 |
| 4 | + 9 | +2 |
| 5 | + 7 | +6 |
| 7 | + 9 | 0 |
| 9 | +10 | +1 |
| 10 | +12 | -1 |
| Note: For each attribute, the above scores are derived by subtracting the mean Magnitude Estimate for each shampoo without the polyacrylamide ingredient, from that for the same shampoo where the polyacrylamide ingredient is present. | | |

It can be deduced from these results that in all the Example shampoos tested, creaminess of foam was improved by the presence of the polyacrylamide. Also, ease of rinsing was not reduced (score = 0) or only very slightly so (score = -1), even though foam creaminess was significantly increased. In certain Examples showing a positive score (+1, +2 and +6) ease of rinsing was improved.

## Claims

**1.** An aqueous shampoo comprising, in addition to water:

a) from 2 to 40% by weight of detergent active compound;

b) from 0.001 to 0.5% by weight of a polyacrylamide having a molecular weight of at least $1 \times 10^5$; and

c) a second polymer chosen from cationic polymers, nonionic polysaccharides and mixtures thereof,

with the proviso that if the polyacrylamide has a molecular weight in the range from $1 \times 10^5$ to $8 \times 10^6$, then the second polymer is a polymer or mixture of polymers chosen from: polygalactomannan gums, polyglycol-polyamine condensation resins, poly(dimethyldiallyl ammonium choride) polymers, hydroxyethylcelluloses, hydroxypropylmethyl celluloses, cationic cellulose ether polymers having the structure:

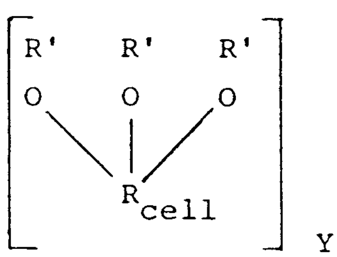

wherein $R_{cell}$ represents the residue of an anhydroglucose unit, Y is an integer of from 50 to 20,000 and wherein each R' individually represents a substituent group of the following general formula:

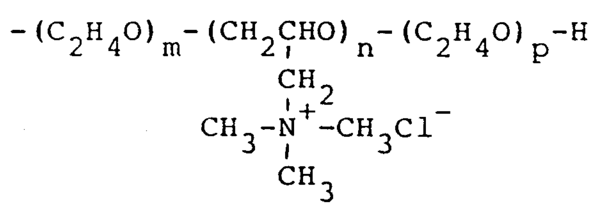

where m is O, or an integer of from 1 to 10, n is O, or an integer of from 1 to 3, and p is O, or an

integer of from 1 to 10, the average value of n per anhydroglucose unit being from 0.35 to 0.45 and the average value of the sum of m and p, per anhydroglucose unit being from 1 to 2;

the amount of the second polymer being from 0.05 to 5% by weight if the second polymer is a polyglycol-polyamine condensation resin, from 0.05 to 3% by weight if the second polymer is poly-(dimethylkdiallyl ammonium chloride) and otherwise from 0.05 to 2% by weight.

**2.** A shampoo according to claim 1, in which the detergent active compound comprises anionic detergent active compound.

**3.** A shampoo according to claim 2, in which the anionic detergent active compound is chosen from $C_{10}$ to $C_{18}$ alkyl sulphates, $C_{10}$ to $C_{18}$ alkyl ether sulphates containing from 1 to 5 moles of ethylene oxide and mixtures thereof.

**4.** A shampoo according to claim 2 or 3, in which the anionic detergent active compound is chosen from sodium lauryl sulphate, ammonium lauryl sulphate, mono-, di- and tri- ethanolammonium lauryl sulphates, sodium lauryl ether sulphate (2EO), sodium lauryl ether sulphate (3EO), potassium lauryl ether sulphate (2EO), ammonium lauryl ether sulphate (3EO) and mixtures thereof.

**5.** A shampoo according to claim 2, in which the anionic detergent active compound is a dialkyl sulphosuccinate having the structure:

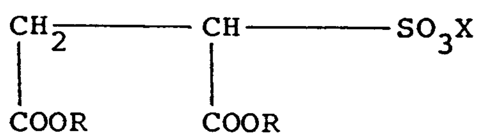

where R represents the same or different straight chain or branched chain alkyl groups having from 4 to 12 carbon atoms; and
X is a solubilising cation chosen from alkali metals, ammonium, substituted ammonium and magnesium.

**6.** A shampoo according to claim 2, in which the anionic detergent active compound is chosen from $\alpha$ -olefin sulphonates, alkyl sarcosinates, alkyl monoglyceride sulphates, alkyl monoglyceride sulphonates, alkyl benzene sulphonates, monoalkylether sulphosuccinates, alkyl ether carboxylates, acyl isethionates and acyl methyl taurides and mixtures thereof.

**7.** A shampoo according to claim 1 in which the detergent active compound comprises an amphoteric detergent active compound chosen from amine oxides, betaines, sulphobetaines, imidazolines and mixtures thereof.

**8.** A shampoo according to any preceding claim, in which the polyacrylamide has a molecular weight of from $1 \times 10^6$ to $1 \times 10^8$.

**9.** A shampoo according to any preceding claim, in which the polyacrylamide has a molecular weight of from $8 \times 10^6$ to $15 \times 10^6$.

**10.** A shampoo according to any preceding claim, in which the polyacrylamide forms from 0.005 to 0.2% by weight of the shampoo.

**11.** A shampoo according to any preceding claim, in which the second polymer is chosen from polygalactomannan gums, cellulosic polymers, polyglycol-polyamine condensation resins, poly-(dimethyldiallylammonium chloride) polymers and mixtures thereof.

**12.** A shampoo according to claim 11, in which the polygalactomannan gum is chosen from hydroxypropyltrimethyl ammonium guar gums, hydroxypropyl guar gums, and mixtures thereof.

**13.** A shampoo according to any of claims 1 to 11, in which the second polymer is chosen from hydroxyethyl celluloses, hydroxypropylmethyl celluloses and mixtures thereof.

**14.** A shampoo according to any preceding claim, in which the second polymer forms from 0.05 to 2% by weight of the shampoo.

**15.** A shampoo according to any preceding claim, in which the second polymer forms from 0.05 to 1% by weight of the shampoo.

**16.** A shampoo according to any preceding claim, which further comprises a phosphate ester having the structure:

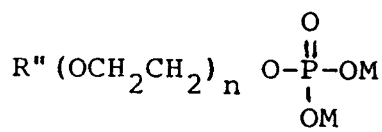

and/or a diester of the structure:

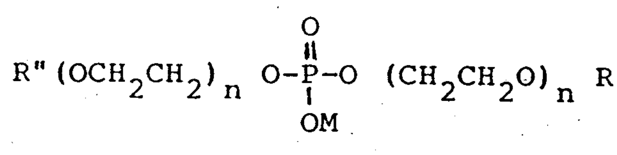

where

| | |
|---|---|
| R" | is an hydrocarbon group having from 8 to 22 carbon atoms; |
| n | is 0 or an integer of from 1 to 10; and |
| M | is hydrogen or an alkali metal, ammonium or amine salt forming group. |

**Revendications**

**1.** Shampooing aqueux comprenant, en plus de l'eau :
a) de 2 à 40% en poids d'un composé détergent actif,
(b) de 0,001 à 0,5% en poids d'un polysaccharide ayant une masse moléculaire d'au moins 1 x $10^5$ ; et
(c) un second polymère choisi parmi les polymères cationiques, des polysaccharides non ioniques et des mélanges de ceux-ci, à la condition que si le polyacrylamide présente une masse moléculaire comprise entre 1 x $10^5$ et 8 x $10^6$, le second polymère soit un polymère ou un mélange de polymères choisi parmi les gommes de polygalactomannane, les résines de condensation de polyglycol et de polyamine, le poly(chlorure de diméthyldiallylammonium), les hydroxyéthylcelluloses, les hydroxypropylméthylcelluloses, les polymères cationiques d'éthers de cellulose de structure :

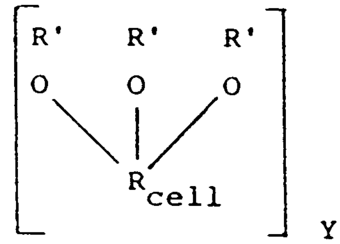

dans laquelle $R_{cell}$ représente le reste d'un motif d'anhydroglucose, Y est un nombre entier de 50 à 20000 et chaque R' représente individuellement un groupe substituant de formule générale suivante

:

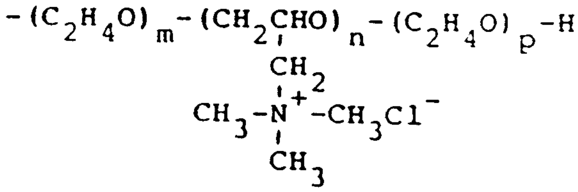

dans laquelle m est 0 ou un nombre entier de 1 à 10, n est 0 ou un nombre entier de 1 à 3 et p est 0 ou un nombre entier de 1 à 10, la valeur moyenne de n par motif d'anhydroglucose étant comprise entre 0,35 et 0,45 et la valeur moyenne de la somme de m + p, par motif d'anhydroglucose étant de 1 à 2 ;
la quantité du second polymère étant comprise entre 0,05 et 5% en poids si le second polymère est une résine de condensation de polyglycol et de polyamine, de 0,05 à 3% en poids si le second polymère est un poly(chlorure de diméthyldiallylammonium) et autrement de 0,05 à 2% en poids.

**2.** Shampooing selon la revendication 1, dans lequel le composé détergent actif comprend un composé détergent actif anionique.

**3.** Shampooing selon la revendication 2, dans lequel le composé détergent actif anionique est choisi parmi les alkylsulfates en $C_{10-18}$, les alkyléthersulfates en $C_{10-18}$ contenant de 1 à 5 moles d'oxyde d'éthylène et leurs mélanges.

**4.** Shampooing selon la revendication 2 ou 3, dans lequel le composé détergent actif anionique est choisi parmi le laurylsulfate de sodium, le lauryl sulfate d'ammonium, les laurylsulfates de mono-, di- et triéthanolammonium, le lauryléthersulfate de sodium (2 0E), le lauryléthersulfate de sodium (3 0E), le lauryléthersulfate de potassium (2 0E), le lauryléthersulfate d'ammonium (3 0E) et leurs mélanges.

**5.** Shampooing selon la revendication 2, dans lequel le composé détergent anionique est un dialkylsulfosuccinate de structure :

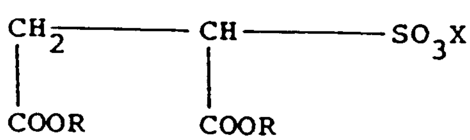

dans laquelle R représente des radicaux alkyle identiques ou différents à chaîne droite ou ramifiée contenant de 4 à 12 atomes de carbone ;
X est un cation solubilisant choisi parmi les métaux alcalins, l'ammonium, l'ammonium substitué et le magnésium.

**6.** Shampooing selon la revendication 2, dans lequel le composé détergent actif anionique est choisi parmi les alpha-oléfinosulfonates, les alkyl-sarcosinates, les alkyl-monoglycéridesulfates, les alkylmonoglycéride sulfonates, les alkylbenzènesulfonates, les monoalkyléthersulfosuccinates, les alkyléthercarboxylates, les acyliséthionates, les acylméthyltaurides et les mélanges de ceux-ci.

**7.** Shampooing selon la revendication 1, dans lequel le composé détergent actif comprend un composé détergent actif amphotère choisi parmi les oxydes d'amines, les bétaïnes, les sulfobétaïnes, les imidazolines et leurs mélanges.

**8.** Shampooing selon l'une quelconque des revendications précédentes, dans lequel la masse moléculaire du polyacrylamide est comprise entre $1 \times 10^6$ et $1 \times 10^8$.

**9.** Shampooing selon l'une quelconque dans lequel la masse moléculaire du polyacrylamide est comprise entre $8 \times 10^6$ et $15 \times 10^6$.

**10.** Shampooing sel on l'une quelconque des revendications précédentes, dans lequel le polyacrylamide constitue de 0,005 à 0,2% en poids du shampooing.

**11.** Shampooing selon l'une quelconque des revendications précédentes, dans lequel le second polymère est choisi parmi les gommes de polygalactomannanes, les polymères cellulosiques, les résines de condensation polyglycol-polyamine, les poly(chlorures de diméthylallylammonium) et leurs mélanges.

**12.** Shampooing selon la revendication 11, dans lequel la gomme de polygalactomannane est choisie parmi les gommes guar d'hydroxypropyltriméthylammonium, les gommes guar d'hydroxypropyle et leurs mélanges.

**13.** Shampooing selon l'une quelconque des revendications 1 à 11, dans lequel le second polymère est choisi parmi les hydroxyéthylcelluloses, les hydroxypropylméthylcelluloses et leurs mélanges.

**14.** Shampooing selon l'une quelconque des revendications précédentes, dans lequel le second polymère constitue de 0,05 à 2% en poids du shampooing.

**15.** Shampooing selon l'une quelconque des revendications précédentes, dans lequel le second polymère constitue de 0,05 à 1% en poids du shampooing.

**16.** Shampooing selon l'une quelconque des revendications précédents, qui comprend en outre un phosphate de structure :

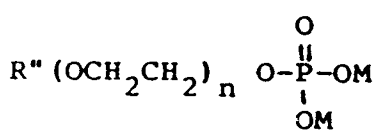

et/ou un diester de structure:

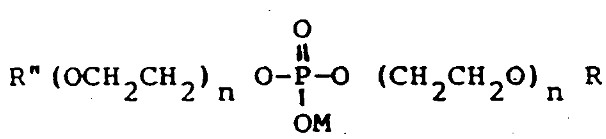

dans lesquelles R'' est un radical hydrocarboné de 8 à 22 atomes de carbone ;
n est 0 ou un nombre entier de 1 à 10 ; et
M est un atome d'hydrogène ou de métal alcalin, l'ammonium ou un groupe formant un sel d'amine.

## Patentansprüche

**1.** Wäßriges Shampoo, umfassend zusätzlich zu Wasser:

a) 2 bis 40 Gew.-% einer detergensaktiven Verbindung;
b) 0,001 bis 0,5 Gew.-% Polyacrylamid mit einem Molekulargewicht von mindestens $1x10^5$ und
c) ein zweites Polymer, ausgewählt aus kationischen Polymeren, nichtionischen Polysacchariden und Mischungen davon,

mit dem Vorbehalt, daß dann, wenn das Polyacrylamid ein Molekulargewicht im Bereich von $1x10^5$ bis $8x10^6$ hat, das zweite Polymer ein Polymer oder eine Mischung von Polymeren ist, ausgewählt aus: Polygalactomannangummis, Polyglycol-Polyamin-Kondensationsharzen, Poly-(dimethyldiallylammoniumchlorid)polymeren, Hydroxyethylcellulosen, Hydroxypropylmethylcellulosen, kationischen Cellulose-Ether-Polymeren mit der Struktur:

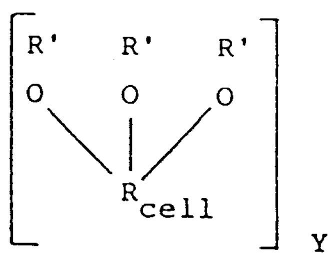

worin $R_{cell}$ den Rest einer Anhydro-Glucose-Einheit darstellt, Y eine ganze Zahl von 50 bis 2000 ist und worin jeder Rest R' unabhängig eine Substituentengruppe der folgenden allgemeinen Formel:

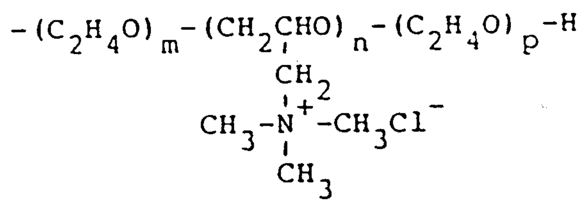

darstellt, worin m 0 oder eine ganze Zahl von 1 bis 10 ist, n 0 oder eine ganze Zahl von 1 bis 3 ist und p 0 oder eine ganze Zahl von 1 bis 10 ist, wobei der Durchschnittswert von n pro Anhydro-Glucose-Einheit 0,35 bis 0,45 ist und der Durchschnittswert der Summe von n und p pro Anhydro-Glucose-Einheit 1 bis 2 ist; die Menge des zweiten Polymers 0,05 bis 5 Gew.-% beträgt, wenn das zweite Polymer ein Polyglycolpolyamin-Kondensationsharz ist, 0,05 bis 3 Gew.-% beträgt, wenn das zweite Polymer Poly(dimethyldiallylammoniumchlorid) ist und ansonsten 0,05 bis 2 Gew.-% ist.

**2.** Shampoo nach Anspruch 1, worin die detergensaktive Verbindung eine anionische detergensaktive Verbindung umfaßt.

**3.** Shampoo nach Anspruch 2, worin die anionische detergensaktive Verbindung ausgewählt ist aus $C_{10}$- bis $C_{18}$-Alkylsulfaten, $C_{10}$- bis $C_{18}$-Alkylethersulfaten, die 1 bis 5 Mol Ethylenoxid enthalten und Mischungen davon.

**4.** Shampoo nach Anspruch 2 oder 3, worin die anionische detergensaktive Verbindung ausgewählt ist aus Natriumlaurylsulfat, Ammoniumlaurylsulfat, Mono-, Di- und Triethanolammonium-Laurylsulfaten, Natriumlaurylethersulfat (2EO), Natriumlaurylethersulfat (3EO), Kaliumlaurylethersulfat (2EO), Ammoniumlaurylethersulfat (3EO) und Mischungen davon.

**5.** Shampoo nach Anspruch 2, worin die anionische detergensaktive Verbindung ein Dialkylsulfosuccinat mit der Struktur:

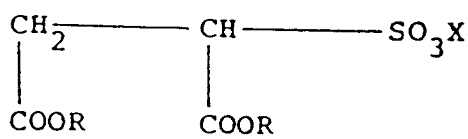

ist, worin R gleiche oder verschiedene geradkettige oder verzweigtkettige Alkylgruppen mit 4 bis 12 Kohlenstoffatomen darstellt und X ein solubilisierendes Kation ist, ausgewählt aus Alkalimetallen, Ammoniumresten, substituierten Ammoniumresten und Magnesium.

**6.** Shampoo nach Anspruch 2, worin die anionische detergensaktive Verbindung ausgewählt ist aus alpha-Olefinsulfonaten, Alkylsarcosinaten, Alkylmonoglyceridsulfaten, Alkylmonoglyceridsulfonaten, Alkylbenzolsulfonaten, Monoalkylethersulfosuccinaten, Alkylethercarboxylaten, Acylisethionaten und Acylmethyltauriden und Mischungen davon.

7. Shampoo nach Anspruch 1, worin die detergensaktive Verbindung eine amphotere detergensaktive Verbindung umfaßt, ausgewählt aus Aminoxiden, Betainen, Sulfobetainen, Imidazolinen und Mischungen davon.

8. Shampoo nach einem der vorhergehenden Ansprüche, worin das Polyacrylamid ein Molekulargewicht von $1x10^6$ bis $1x10^8$ hat.

9. Shampoo nach einem der vorhergehenden Ansprüche, worin das Polyacrylamid ein Molekulargewicht von $8x10^6$ bis $15x10^6$ hat.

10. Shampoo nach einem der vorhergehenden Ansprüche, worin das Polyacrylamid 0,005 bis 0,2 Gew.-% des Shampoos bildet.

11. Shampoo nach einem der vorhergehenden Ansprüche, worin das zweite Polymer ausgewählt ist aus Polygalactomannangummis, Cellulosepolymeren, Polyglycolpolyamin-Kondensationsharzen, Poly-(dimethyldiallylammoniumchlorid)polymeren und Mischungen davon.

12. Shampoo nach Anspruch 11, worin der Polygalactomannangummi ausgewählt ist aus Hydroxypropyltrimethylammoniumguargummi, Hydroxypropylguargummi und Mischungen davon.

13. Shampoo nach einem der Ansprüche 1 bis 11, worin das zweite Polymer ausgewählt ist aus Hydroxyethylcellulosen, Hydroxypropylmethyl-Cellulosen und Mischungen davon.

14. Shampoo nach einem der vorhergehenden Ansprüche, worin das zweite Polymer 0,05 bis 2 Gew.-% des Shampoos bildet.

15. Shampoo nach einem der vorhergehenden Ansprüche, worin das zweite Polymer 0,05 bis 1 Gew.-% des Shampoos bildet.

16. Shampoo nach einem der vorhergehenden Ansprüche, das weiterhin einen Phosphatester mit der Struktur:

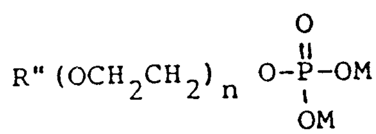

und/oder einen Diester der Struktur

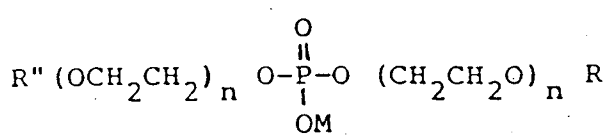

umfaßt, worin R" eine Kohlenwasserstoffgruppe mit 8 bis 22 Kohlenstoffatomen ist; n 0 oder eine ganze Zahl von 1 bis 10 ist und M Wasserstoff oder ein Alkalimetall, eine Ammonium- oder Aminsalz bildende Gruppe ist.